# EUROPEAN PATENT SPECIFICATION

(11) **EP 4 059 948 B1**
(45) Date of publication and mention of the grant of the patent: **23.09.2026**
(21) Application number: 20888429.6
(22) Date of filing: 13.11.2020
(51) Int. Cl.: C12Q 1/6806, C12N 5/00, C12N 9/22, C12N 15/113, C07K 14/005, C07K 14/705, C40B 40/06

(54) **LIBRARY OF BARCODED EXTRACELLULAR VESICLES**
BIBLIOTHEK VON STRICHCODIERTEN EXTRAZELLULÄREN VESIKELN
BANQUE DE VÉSICULES EXTRACELLULAIRES À CODE-BARRES

(30) Priority: 15.11.2019 JP 2019207329
(43) Date of publication of application: 21.09.2022
(73) Proprietor: The University of Tokyo, Bunkyo-ku, Tokyo 113-8654 (JP)
(72) Inventor: KOJIMA Ryosuke, Tokyo 113-8654 (JP); KUNITAKE Koki, Tokyo 113-8654 (JP); FURUTSUKI Sei, Tokyo 113-8654 (JP); URANO Yasuteru, Tokyo 113-8654 (JP)
(74) Representative: Michalski Hüttermann & Partner mbB
(86) International application number: PCT/JP2020/042416
(87) International publication number: WO 2021/095842

(56) References cited:
- WO-A1-2019/199941
- GB-A- 2 568 255
- JP-A- 2014 512 826
- JP-A- 2016 533 164
- US-A1- 2017 087 087
- US-A1- 2017 159 136
- US-A1- 2019 085 383
- LU ALBERT ET AL: "Genome-wide interrogation of extracellular vesicle biology using barcoded miRNAs", ELIFE, vol. 7, 17 December 2018 (2018-12-17), pages 1 - 23, XP055823537, DOI: 10.7554/eLife.41460
- KOJIMA RYOSUKE ET AL: "Designer exosomes produced by implanted cells intracerebrally deliver therapeutic cargo for Parkinson's disease treatment", NATURE COMMUNICATIONS, vol. 9, no. 1, 3 April 2018 (2018-04-03), pages 1305, XP055823536, DOI: 10.1038/s41467-018-03733-8
- DI WU ET AL: "Profiling surface proteins on individual exosomes using a proximity barcoding assay", NATURE COMMUNICATIONS, vol. 10, no. 1, 26 August 2019 (2019-08-26), XP055767129, DOI: 10.1038/s41467-019-11486-1
- KOJIMA R. ET AL.: "Designer exosomes produced by implanted cells intracerebrally deliver therapeutic cargo for Parkinson' s disease treatment", NATURE COMMUNICATIONS, vol. 9, no. 1, 3 April 2018 (2018-04-03), pages 1305, XP055823536, DOI: 10.1038/s41467-018-03733-8
- LU A. ET AL.: "Genome-wide interrogation of extracellular vesicle biology using barcoded miRNAs", ELIFE, vol. 7, 17 December 2018 (2018-12-17), pages e41460, XP055823537, DOI: doi.org/10.7554/eLife.41460.001
- BRYANT K.L. ET AL.: "A novel fluorescence-based biosynthetic trafficking method provides pharmacologic evidence that PI4-kinase IIIalpha is important for protein trafficking from the endoplasmic reticulum to the plasma membrane", BMC CELL BIOLOGY, vol. 16, no. 1, 27 February 2015 (2015-02-27), pages 5, XP021213586, DOI: 10.1186/ s12860-015-0049-5
- KAMMULA E.C. ET AL.: "Brain Transcriptome-Wide Screen for HIV-1 Nef Protein Interaction Partners Reveals Various Membrane- Associated Proteins", PLOS ONE, vol. 7, no. 12, 17 December 2012 (2012-12-17), pages e51578, XP055823538
- MIRANDA A.M. ET AL.: "Neuronal lysosomal dysfunction releases exosomes harboring APP C-terminal fragments and unique lipid signatures", NATURE COMMUNICATIONS, vol. 9, no. 1, 18 January 2018 (2018-01-18), pages 291, XP055823540, DOI: 10.1038/s41467-017-02533- w
- ICHIOKA F. ET AL.: "HD-PTP and Alix share some membrane-traffic related proteins that interact with their Brol domains or proline-rich regions", ARCHIVES OF BIOCHEMISTRY AND BIOPHYSICS, vol. 457, no. 2, 2007, pages 142 - 149, XP005762557, DOI: 10.1016/j.abb.2006.11.008
- STEIN K. ET AL.: "Vps15p regulates the distribution of cup-shaped organelles containing the major eisosome protein Pillp to the extracellular fraction required for endocytosis of extracellular vesicles carrying metabolic enzymes", BIOLOGY OF THE CELL, vol. 109, no. 5, May 2017 (2017-05-01), pages 190 - 209, XP055823542
- JONCKHEERE V. ET AL.: "Omics Assisted N-terminal Proteoform and Protein Expression Profiling on Methionine Aminopeptidase 1 (MetAP1) Deletion", MOLECULAR & CELLULAR PROTEOMICS, vol. 17, April 2018 (2018-04-01), pages 694 - 708, XP055823543
- SAKAMOTO, OSAMU: "III. Organic acid and fatty acid metabolic abnormality. Methylmalonic acidemia. Adenosylcobalamin synthesis defect. 2nd ed.", pages: 356 - 359
- TAKAHASHI-INIGUEZ T. ET AL.: "Human MMAA induces the release of inactive cofactor and restores methylmalonyl-CoA mutase activity through their complex formation", BIOCHIMIE, vol. 142, 2017, pages 191 - 196, XP085244567, DOI: 10.1016/j.biochi.2017.09.012
- CHEN R. ET AL.: "Friend or Evidence Indicates Endogenous Exosomes Can Deliver Functional gRNA and Cas9 Protein", SMALL, vol. 15, no. 38, 18 September 2019 (2019-09-18), pages 1902686, XP055823545, DOI: 10.1002/smll.201902686
- JOUNG J. ET AL.: "Genome-scale CRISPR-Cas9 knockout and transcriptional activation screening", NATURE PROTOCOLS, vol. 12, no. 4, 2017, pages 828 - 863, XP037473979, DOI: 10.1038/nprot.2017.016
- ANONYMOUS: "CRISPR Activation/Interference", AGILENT, 1 January 2018 (2018-01-01), XP055823547, Retrieved from the Internet <URL:https://www.chem-agilent.com/pdf/low_5991-9161JAJP.pdf> [retrieved on 20201224]
- KUNITAKE, KOKI : "27M-aml12S Comprehensive screen of regulator of exosome secretion using RNA barcodes", LECTURE ABSTRACTS OF THE 140TH ANNUAL MEETING OF THE PHARMACEUTICAL SOCIETY OF JAPAN; KYOTO; MARCH 25-28, 2020, vol. 140, 1 January 2020 (2020-01-01) - 28 March 2020 (2020-03-28), pages 27M - am12S, XP009537636
- KUNITAKE, KOKI: "4P-0621 Comprehensive screen of regulator of exosome secretion using RNA barcodes", 42ND ANNUAL MEETING OF THE MOLECULAR BIOLOGY SOCIETY OF JAPAN; FUKUOKA; DECEMBER 3-6, 2019, vol. 42, 19 November 2019 (2019-11-19) - 6 December 2019 (2019-12-06), pages 4P - 0621, XP009537477

## Description

### Technical Field

### <Cross Reference>

This application claims the benefit of priority from a Japanese patent application (Japanese Patent Application No. 2019-207329, having the publication number JP2025128161A) filed November 15, 2019.

The present invention relates to a library of barcoded extracellular vesicles.

### Background Art

It has been clarified that there are several types of vesicles (extracellular vesicles: EVs) to be secreted from cells depending on their origin and characteristics. There are a large number of classification methods based on heterogeneities, and from the viewpoint of size, EVs can be roughly divided into EVs with a diameter of 200 nm or less (small EVs) and large EVs larger than them. The former is membrane vesicles with a diameter of about 30 to 200 nm, and the membrane thereof comprises a large amount of exosomes that are thought to be mainly derived from endosomes.

Exosomes also have heterogeneity, but it is thought that many of them plentifully comprise endosome-binding proteins, such as RabGTPase, SNARE, annexin, and phloretin, and tetraspanins (CD63, CD81, CD9, etc.) which are a transmembrane protein family. The large EV comprises, as main constitutional components of the cell membrane, for example, a microvesicle (MV, or also referred to as ectosome) which is thought to be a main component of membrane and an apoptotic body formed by fragmentation of a cell on apoptosis (PTLs 1, 2, and 3).

MV is formed by being directly torn off from cell membrane and is a vesicle having a size of about 200 nm to 1000 nm. MV comprises an integrin, a selectin, CD40, etc.

An apoptotic body is also similarly formed by being directly torn off from cell membrane, but is a vesicle having a size of 500 nm to 2000 nm and contains a fragmented genomic DNA, a histone protein, etc.

It has been reported that exosomes are involved in short-distance or long-distance intercellular communication.

For example, in an immune system, an exosome released from a cell functions as an antigen-presenting vesicle and induces, for example, response in antitumor immunity and immune tolerance to suppress inflammation (NPL 4).

In neurodegenerative diseases, it is known that a pathogenic protein, such as prion and β-amyloid peptide, uses an exosome when it propagates to another cell.

An exosome is low in immunogenicity, has characteristics, such as passing through the blood brain barrier, and comprises various nucleic acids (such as an mRNA, an miRNA, an shRNA, and an ncRNA), in addition to proteins, in the inside thereof. It is known that these nucleic acids function in an exosome-receiving cell and influence the function of the exosome-receiving cell.

Accordingly, in recent years, studies using exosomes in a DDS (drug delivery system) are being actively carried out with expectation of therapeutic effects by functional modification of exosome-receiving cells (NPL 5).

For example, enhancement of the efficiency of targeting to exosome-receiving cells by expressing a peptide or protein recognized by the exosome-receiving cells on the membrane surface of an exosome and also an attempt of increasing the efficiency of the DDS by expressing an RNA-binding protein on the exosome intima side and efficiently recruiting the RNA of cytoplasm have been performed (NPLs 6 to 9 and PTL 1).

The present inventors also have developed exosomes suitable for a DDS (NPL 10).

In addition, it has been advocated that a cancer cell programs a metastasis site by an exosome secreted by the cancer cell itself to construct an environment advantageous for its own metastasis. Therefore, there are also papers suggesting that if secretion of exosomes from cancer cells can be selectively inhibited, it is possible to develop an anticancer agent (NPLs 11 and 12). They suggest that there is a relationship between the type of an integrin existing on the exosome surface and the type of an organ to which cancer metastasizes.

It has been suggested that extracellular vesicles not only are those characteristic to animals but also exist in plants and play an important role in biological defense (plant immunity) against pathogens and so on, but the functions of extracellular vesicles are not well known (NPL 13).

Albert et al. (2018), "Genome-wide interrogation of extracellular vesicle biology using barcoded miRNAs", eLife, vol. 7, 17 December 2018 (2018-12-17), pages 1-23, discloses barcoded, exosomal microRNAs (bEXOmiRs) to monitor extracellular vesicle release quantitatively using deep sequencing. Albert et al. does not disclose extracellular vesicles comprising a fusion protein and a barcode RNA bound to the fusion protein. Ryosuke et al. (2018), "Designer exosomes produced by implanted cells intracerebrally deliver therapeutic cargo for Parkinson's disease treatment", Nature Communications, vol. 9, no. 1, 3 April 2018 (2018-04-03), page 1305, discloses an active packaging device of specific RNAs into exosomes and a method for screening cells to find genes that enhance exosome production. Ryosuke et al. does not disclose the use of a barcode RNA bound to the fusion protein, the fusion protein comprising a protein existing in an extracellular vesicle and an RNA-binding protein.

US2017/149136A1 discloses RNA binding domains specifically fused to vesicle-specific and/or exosome-specific proteins to track multiple vesicles and/or exosomes to their originating cells. US2017/149136A1 does not disclose a method of screening for a factor that affects stability of an extracellular vesicle in body fluid.

US2017/087087A1 discloses packaging of cargo RNA comprising the MS2 RNA packaging signal into exosomes in the presence of a fusion protein comprising the MS2 coat protein RNA-binding domain. US2017/087087A1 does not disclose the use of barcoded RNAs for screening for a factor that affects kinetics and/or stability of an extracellular vesicle in tissue or body fluid.

WO2019/199941A1 discloses extracellular vesicles, wherein the cargo RNA comprises an RNA-motif and the RNA-binding domain of the fusion protein binds specifically to the RNA-motif of the cargo RNA. WO2019/199941A1 does not disclose the use of barcoded RNAs.

GB2568255A discloses amnion cells engineered to express the shRNA and fusion polypeptide constructs comprising as nucleic acid-binding domain either PUF (obtained from human PUM protein) or Cas6, combined with CD63 or CD81 as the EV polypeptides, respectively. GB2568255A does not disclose the use of barcoded RNAs.

Wu et al. (2019), "Profiling surface proteins on individual exosomes using a proximity barcoding assay", Nature communications, discloses a proximity-dependent barcoding assay (PBA), as a high-throughput approach to simultaneously profile 38 surface proteins for their presence on individual exosomes. Wu et al. does not disclose a fusion protein comprising a protein existing in an extracellular vesicle and an RNA-binding protein.

### Citation List

### Patent Literature

PTL 1: U.S. Patent Publication No. 2015/0093433

### Non Patent Literature

NPL 1: eLife (2018); 7: e41460
NPL 2: Journal of Extracellular Vesicles (2015), 4, 26316
NPL 3: Journal of Extracellular Vesicles (2018), 7, 1535750
NPL 4: Immunological Reviews (2013), Vol. 251, pp. 125-142
NPL 5: NATURE (2017), Vol. 546, pp. 498-521
NPL 6: Nano letters (2019), 19, 19-28
NPL 7: Journal of Extracellular Vesicles (2016), 5, 31027
NPL 8: Journal of Extracellular Vesicles (2016), 5, 31053
NPL 9: Nature Biotechnology (2011), 29, 341-345
NPL 10: NATURE COMMUNICATIONS (2018), 9, 1305
NPL 11: NATURE (2015), Vol. 527 (7578), pp. 329-35
NPL 12: Sci. Rep., (2018), 8, 8161
NPL 13: Plant Physiology (2017), Vol. 173, pp. 728-741
NPL 14: NATURE COMMUNICATIONS (2017), 8, 15178

### Summary of Invention

### Technical Problem

It is an object of the present invention to provide a library of barcoded extracellular vesicles, a method for manufacturing it, and a method for using it.

### Solution to Problem

As a result of diligent research, the present inventors succeeded in creating a library of extracellular vesicles containing nucleic acids for comprehensively screening factors that influence the properties of extracellular vesicles (i.e., pharmacokinetics of an extracellular vesicle, secretion level of an extracellular vesicle, the contained substances (e.g., protein and nucleic acid), and the composition of extracellular vesicle membrane (e.g., phospholipid constituting the membrane and protein localized in the extracellular vesicle membrane).

The invention is defined by the features of the appended independent claims. Preferred embodiments are defined by the features of the dependent claims.

In particular, the present invention relates to a method of screening for a factor that contributes to stability of an extracellular vesicle in body fluid or a factor that promotes or inhibits secretion of an extracellular vesicle into body fluid,the method comprising;
(1) a step of providing a library comprising multiple types of extracellular vesicles comprising a fusion protein and a barcode RNA bound to the fusion protein, the fusion protein comprising a protein existing in an extracellular vesicle and an RNA-binding protein and the barcode RNA comprising a recognition sequence for the RNA-binding protein; and
(2) a step of detecting barcode RNAs from the extracted RNAs from a body fluid sample obtained from a subject administered with the library comprising multiple types of extracellular vesicles from step 1.

The present invention further relates to a method of screening for a factor that influences efficiency of targeting of an extracellular vesicle to tissue or body fluid, the method comprising:
(1) a step of providing a library comprising multiple types of extracellular vesicles comprising a fusion protein and a barcode RNA bound to the fusion protein, the fusion protein comprising a protein existing in an extracellular vesicle and an RNA-binding protein and the barcode RNA comprising a recognition sequence for the RNA-binding protein; and
(2) a step of detecting barcode RNAs from the extracted RNAs from a tissue sample or body fluid sample obtained from a subject administered with the library comprising multiple types of extracellular vesicles from step 1.

The present invention further relates to an in vitro method of screening for a factor that influences efficiency of targeting of an extracellular vesicle to a cell, the method comprising:
(1) a step of providing multiple types of extracellular vesicles comprising a fusion protein and s barcode RNA bound to the fusion protein, the fusion protein comprising a protein existing in an extracellular vesicle and an RNA-binding protein and the barcode RNA comprising a recognition sequence for the RNA-binding protein;
(2) a step of administering the multiple types of extracellular vesicles to a cell;
(3) a step of extracting RNAs from the cell; and
(4) a step of detecting barcode RNAs from the extracted RNAs.

### Advantageous Effects of Invention

The present invention can be used for developing an efficient drug delivery system using an extracellular vesicle, biology research for extracellular vesicles, and drug discovery research targeting an extracellular vesicle secretory path.

### Brief Description of Drawings

[Fig. 1] Fig. 1 shows evaluation of barcoded CD63-MS2-expressing EVs.
[Fig. 2] Fig. 2 shows a CD63-MS2-expressing EV library (for gRNAs and for random peptide-Lamp2b) and a CD63-dCas9-expressing EV library.
[Fig. 3] Fig. 3 shows gRNAs of which the component ratios in blood are significantly increased;
[Fig. 4] Fig. 4 shows screening (1) for a factor that promotes or inhibits secretion of an EV using bar barcoded EVs.
[Fig. 5] Fig. 5 shows screening (2) for a factor that promotes or inhibits secretion of an EV using bar barcoded EVs.
[Fig. 6] Fig. 6 shows an increase in the secretion level of a CD63 positive EV by a PI4KA inhibitor.

### Description of Examples

In the present disclosure, a "comprising" state encompasses an "essentially comprising" state and a "consisting of" state.

Disclosed herein is a nucleic acid encoding a fusion protein comprising a protein existing in an extracellular vesicle and an RNA-binding protein, or a fusion protein comprising a protein existing in an extracellular vesicle and an RNA-binding protein. The extracellular (secretory) vesicle (EV) is a vesicle that is used for releasing an intracellular substance to the outside of the cell and is formed of a phospholipid double layer. Examples of lipid compositions include sphingomyelin and phosphatidylserine. The size thereof is 10 nm to 10 µm, 30 nm to 5000 nm, or 50 nm to 3000 nm in diameter, and a small EV (mainly including an exosome and a microvesicle) having a diameter of 10 nm or more, 20 nm or more, 30 nm or more, 40 nm or more, or 50 nm or more and 500 nm or less, 400 nm or less, 300 nm, or 200 nm or less is suitable in the present disclosure, but not limited thereto. The origin thereof is preferably a eukaryote, but is not particularly limited. Preferred is an extracellular vesicle derived from a human, a non-human mammal (such as a mouse and a rat), a higher plant, or a microorganism (such as an enterobacterium).

In the present disclosure, the protein existing in an extracellular vesicle is preferably a marker of the extracellular vesicle, that is, a protein that demonstrates the presence of a (specific) extracellular vesicle through the detection of the protein (i.e., a protein that abundantly exists in an extracellular vesicle or specifically exists in an extracellular vesicle). The origin thereof is not particularly limited and is preferably derived from a human, a non-human mammal (such as a mouse and a rat), a higher plant, or a microorganism (such as an enterobacterium).

According to NPL 3, the markers of mammalian extracellular vesicles are classified as follows:
The membrane proteins or GPI-anchored proteins that can be used as marker proteins of extracellular vesicles are, for example,
1) Tissue-nonspecific proteins:
   Tetraspanins (CD63, CD9, CD81, and CD82), other multi-pass transmembrane proteins (such as CD47 and heterotrimeric G proteins (GNA: guanine nucleotide-binding proteins));
   MHC class I (HLA-A/B/C and H2-K/D/Q), integrin (ITGA/ITGB), transferrin receptor (TFR2);
   LAMP1/2;
   Heparan sulfate proteoglycan (including Syndecan (SDC));
   Extracellular matrix metalloprotease derivatives (EMMPRIN) (also called BSG or CD147);
   ADAM10;
   GPI-anchored 5'-nucleotide, CD73 (NT5E),
   GPI-anchored complement-binding proteins, CD55 and CD59; and
   Sonic hedgehog protein (SHH);
2) Cell/tissue-specific proteins:
   Several tetraspanins: TSPAN8 (epithelial cell-specific), CD37 and CD53 (leukocyte-specific);
   PECAM1 (endothelial cell-specific);
   ERBB2 (breast cancer-specific);
   EPCAM (epithelial-specific);
   CD90 (THY1) (mesenchymal stem cell-specific);
   CD45 (PTPRC) (immune cell-specific), CD41 (ITGA2B) or CD42a (GP9) (thrombocyte-specific);
   Glycophorin A (GYPA) (erythrocyte-specific);
   CD14 (monocyte-specific), MHC class II (HLA-DR/DP/DQ, H2-A);
   CD3 (T cell-specific);
   Acetylcholine esterase/AChE-S (nerve cell-specific), AChE-E (erythrocyte-specific); and
   Amyloid β A4/APP (nerve cell-specific).
   Cytoplasm proteins that can be used as marker proteins of extracellular vesicles are, for example,
   ESCRT-I/II/III (TSG101 and CHMP) and accessory proteins: ALIX (PDCD6IP) and VPS4A/B;
   ARRDC1;
   Flotillin-1 and 2 (FLOT1/2);
   Caveolin (CAV);
   EHD;
   RHOA;
   Annexin (ANXA);
   Heat shock proteins, HSC70 (HSPA8) and HSP84 (HSP90AB 1);
   ARF6;
   Syntenin (SDCBP); and
   Microtubule-associated protein tau (Tau, MAPT: nerve cell-specific).

In the present disclosure, the protein existing in an extracellular vesicle may be a naturally occurring protein (including polymorphism, orthologue, and paralogue). Alternatively, the protein may be an artificial mutant in which some of the amino acids are added, substituted, or deleted or may be a fragment thereof (e.g., "exoTOPE"), but is preferably an artificial mutant or fragment that does not change the localization of the protein.

The term RNA-binding protein refers to a protein that can bind to an RNA with or without dependence on the sequence of the RNA. As the RNA-binding ability, the dissociation constant (Kd) to an RNA is preferably 1 µM or less, 500 nM or less, 300 nM or less, 100 nM or less, 50 nM or less, 30 nM or less, 10 nM or less, 5 nM or less, 3 nM or less, 1 nM or less, 500 pM or less, 300 pM or less, 100 pM or less, 50 pM or less, 30 pM or less, 10 pM or less, 5 pM or less, or 3 pM or less. Examples of the RNA-binding protein include bacteriophage MS2 coat proteins (maturation gene products) (Kd with an RNA comprising an MS2 recognition sequence is 3 to 300 nM), CAS (CRISPR-associated gene) products (Cas9 (including SpCas9, SaCas9, and a mutant (dCas9) lacking the endonuclease activity, Kd with a gRNA is 10 pM), Cpf1 (such as Cas12a and Cas13), L7Ae, λ bacteriophage antiterminator protein N, and HuR (human antigen R). The RNA-binding protein may be a naturally occurring protein (including polymorphism, orthologue, and paralogue). Alternatively, the protein may be an artificial mutant in which some of the amino acids are added, substituted, or deleted or may be a fragment thereof, but is preferably an (active) artificial mutant or (active) fragment maintaining the RNA-binding ability (it is preferable to have a binding ability of at least 50% or more, 60% or more, 70% or more, 80% or more, 90% or more, or 100% or more of the binding ability of the corresponding natural protein).

In the present disclosure, the fusion protein of a protein existing in an extracellular vesicle and an RNA-binding protein may further comprise a transcription factor, a transcriptional activator (e.g., VP64, p65, Rta, or VPH), a transcriptional repressor (e.g., KRAB), or an active fragment thereof. The fusion protein may be further fused with a marker peptide (e.g., GFP or HisTag).

The fusion protein of a protein existing in an extracellular vesicle and an RNA-binding protein may comprise post-translational modification (e.g., glycosylation or phosphorylation).

Disclosed herein is a nucleic acid that influences a property of an extracellular vesicle. Examples of the nucleic acid that influences a property of an extracellular vesicle include nucleic acids that cause changes when a cell secreting an extracellular vesicle comprises the nucleic acid. When the extracellular vesicle secreted from the cell is analyzed, the changes are, for example, as follows:
(1) the amount of endogenous protein existing in the secreted extracellular vesicle or on the surface thereof changes compared to when the cell secreting the extracellular vesicle does not comprise the nucleic acid;
(2) the secretion level of the extracellular vesicle changes compared to when the cell secreting the extracellular vesicle does not comprise the nucleic acid;
(3) the lipid membrane constituting the extracellular vesicle membrane is influenced compared to when the cell secreting the extracellular vesicle does not comprise the nucleic acid; and
(4) the exogenous protein encoded by the nucleic acid exists in the extracellular vesicle or on the surface thereof.

The nucleic acid may be a naturally occurring DNA or RNA or may be a mixture thereof. The nucleic acid may be one that does not exist in nature (for example, a nucleic acid in which some or all of the nucleotides are S-modified (phosphorothioated) without bonding to each other with a phosphorylation ester (P), or a peptide nucleic acid (PNA)).

Examples of the nucleic acid causing (1) above include nucleic acids encoding proteins, such as a nucleic acid encoding the endogenous protein itself, a nucleic acid encoding a transcription factor that controls the transcription of the endogenous protein, a nucleic acid encoding a factor involved in post-translational modification of the endogenous protein, and a nucleic acid encoding a factor involved in chaperoning (including folding and intracellular transportation) of the endogenous protein; and an antisense RNA (siRNA), miRNA (microRNA), shRNA (small hairpin RNA), and snRNA (small nuclear RNA) that positively or negatively control the expression of the endogenous protein, and a nucleic acid (e.g., gRNA that is used in CRISPR/Cas9 system) for modifying a gene encoding the endogenous protein in genome-editing technology (e.g., ZFN (zinc-finger nuclease), TALEN (transcription activator-like effector nuclease), CRISPR (clustered regularly interspaced short palindromic repeats)/Cas9 (crispr associated protein 9)).

Examples of the nucleic acid causing (2) above include a nucleic acid encoding a factor that influences the secretion level of an extracellular vesicle and a nucleic acid encoding a factor that controls transcription, translation, and expression of a factor influencing the secretion level itself of an extracellular vesicle; and an antisense RNA, miRNA, shRNA, and snRNA that positively or negatively control the expression of a factor that influences the secretion level of an extracellular vesicle or a factor that controls transcription, translation, and expression of a factor influencing the secretion level itself of an extracellular vesicle, and a nucleic acid (e.g., gRNA that is used in CRISPR/Cas9 system) for modifying a gene encoding a factor that influences the secretion level of an extracellular vesicle or a factor that controls the transcription, translation, and expression of a factor influencing the secretion level itself of an extracellular vesicle in genome-editing technology (e.g., ZFN, TALEN, CRISPR/Cas9).

Examples of the nucleic acid causing (3) above include a nucleic acid encoding an enzyme for synthesizing lipid membrane constituting extracellular vesicle membrane, and a nucleic acid encoding a factor that controls the transcription, translation, and expression of an enzyme for synthesizing lipid membrane constituting extracellular vesicle membrane; and an antisense RNA, miRNA, shRNA, and snRNA that negatively or positively control the expression of an enzyme for synthesizing lipid membrane constituting extracellular vesicle membrane or a factor that controls transcription, translation, and expression of an enzyme for synthesizing lipid membrane constituting extracellular vesicle membrane, and a nucleic acid (e.g., gRNA that is used in CLISPR/Cas9 system) for modifying a gene encoding an enzyme for synthesizing lipid membrane constituting extracellular vesicle membrane or a factor that controls transcription, translation, and expression of an enzyme for synthesizing lipid membrane constituting extracellular vesicle membrane in genome-editing technology (e.g., ZFN, TALEN, CRISPR/Cas9).

Examples of the nucleic acid causing (4) above include a nucleic acid encoding an exogenous protein.

In the present disclosure, the nucleic acid that influences a property of an extracellular vesicle may comprise an mRNA or an ncRNA and may further comprise a recognition sequence for an RNA-binding protein.

In the present disclosure, the term mRNA refers to an RNA comprising an RNA (cRNA: coding RNA) having nucleotide sequence information and structure that can be translated into a protein (or peptide), and includes not only a naturally occurring mRNA but also an RNA not having an m7G cap on the 5'-end or an RNA not having a polyadenylated (poly A) 3'-end. A premature mRNA that can become nucleotide sequence information and structure for translation into a protein when appropriate splicing occurs in a cell is also included.

The term ncRNA (non-coding RNA) refers to an RNA (functional nucleic acid) that does not have nucleotide sequence information or structure that can be translated into a protein but have a certain function in vivo. Although there is no particular limitation, a small nuclear RNA (snRNA) and a small nucleolar RNA (snoRNA) that form a complex with a protein in a nucleus and an miRNA (including pre-miRNA) and an siRNA (including pre-siRNA (e.g., shRNA (small hairpin RNA)) that binds to another RNA are included.

Furthermore, the ncRNA may include a guide RNA (gRNA, including a single-stranded guide RNA) (RNA that guides an RNA-protein complex to a target nucleic acid molecule by complementary binding) may be included. In the CRISPR/Cas9 system in bacteria and archaebacteria, two RNAs, i.e., a crRNA (CRISPR RNA) recognizing a target DNA sequence of about 20 nucleotides and a tracrRNA (transactivating crRNA) functioning as a scaffold for binding to Cas9, are compounded to function as a gRNA, but an sgRNA (single guide RNA) in which they are bonded to each other into one RNA for the purpose of genome editing is also included in the gRNA. When Cpf1 is used instead of Cas9, a crRNA of 41 to 44 nucleotides (the recognition region is 21 to 24 nucleotides long) functions alone as a gRNA.

The term recognition sequence for an RNA-binding protein refers to a sequence to which an RNA protein can bind. The RNA protein can bind to the sequence in a monomer state or in a multimer or can bind in a heteromultimeric form with another factor.

For example, in the case of MS2, ACAUGAGGAUCACCCAUGU (SEQ ID NO: 1); in the case of Cas9, CAGCAUAGCAAGUUUAAAUAAGGCUAGUCCGUUAUCAACUUGAAAAAGU GGCACCGAGUCGGUGC (SEQ ID NO: 2) as a tracrRNA; in the case of L7Ae, GGGUACCGUGAUCCGAAAGGUGAGUACCC (SEQ ID NO: 3); in the case of LBAPN (λ bacteriophage antiterminator protein N), GCCCUGAAGAAGGGC (SEQ ID NO: 4); and in the case of HuR, AUUUACCCAUUUACCCAUUUACCCAUUUACCCAUUUACCCAUUUA (SEQ ID NO: 5) can be used as the recognition sequence for an RNA-binding protein, but the sequence is not limited thereto.

The cell that secrets an extracellular vesicle according to the present disclosure is not particularly limited as long as its origin is derived from a eukaryote, and is preferably derived from a human, a non-human animal (such as a mouse or a rat), or a higher plant. The mammalian cell may be a stem cell (including an induced pluripotent stem cell (iPS cell), an embryonic stem cell (ES cell), and a somatic stem cell (including a mesenchymal stem cell, an adipose stem cell, a hematopoietic stem cell, a neural stem cell, a vascular endothelial stem cell, a hepatic stem cell, and an epithelia stem cell)), a cell differentially induced from a stem cell, an epithelial cell, an endothelial cell, a fibroblast cell, a cancer cell, an immune cell (dendritic cell or blood cell), or a nerve cell, or a cell line established from these cells. The cell may be a cultured cell (e.g., HEK293T cell).

Disclosed herein is a method for creating a library of extracellular vesicles comprising barcode RNAs and a screening method using the library. The screening method comprises identification of a specific barcode RNA comprised in an extracellular vesicle.

The barcode RNA is an RNA comprising an mRNA and/or an ncRNA and enables distinguishment of an intracellular vesicle by being comprised in the intracellular vesicle.

The library of extracellular vesicles containing barcode RNAs of the present disclosure comprises at least two or more extracellular vesicles, and the distinguishment of an extracellular vesicle is performed by determining the sequence of a barcode RNA contained therein. The library preferably comprises 5000 or more, 6000 or more, 7000 or more, 8000 or more, 9000 or more, or 10000 or more extracellular vesicles.

The method for introducing a barcode RNA into a cell is not particularly limited, and a barcode RNA itself may be introduced into a cell (for example, using a method such as a microinjection method, an electroporation method, or a gene transfer method using a cationic liposome), or a barcode RNA may be introduced by introducing an expression vector (such as a DNA vector or an RNA vector (including virus vector)) expressing a barcode RNA into a cell and transcribing the barcode RNA in the cell. A library of extracellular vesicles can be obtained by collecting extracellular vesicles secreted from cells comprising barcode RNAs. For example, the secreted extracellular vesicles can be collected from the cell culture supernatant.

In addition, when extracellular vesicles are collected, a specific type of extracellular vesicle only may be collected by using the marker of the specific extracellular vesicle as an indicator. For example, by using an antibody that recognizes a membrane protein, such as a tetraspanin, existing in the membrane of an extracellular vesicle, it is possible to collect only an extracellular vesicle comprising the tetraspanin on the membrane surface without breaking the extracellular vesicle. Barcode RNAs are collected from cells, tissue, body fluid, or extracellular vesicles, and sequences thereof are determined. In the case of tissue or body fluid, extracellular vesicles are isolated from them once, and barcode RNAs may be collected from the isolated extracellular vesicles. The extraction method is not particularly limited, but is preferably performed using an RNA isolation reagent, such as a TRIzol reagent mainly composed of phenol and guanidine isothiocyanate, or a generic RNA purification kit.

Preferably, the collected barcode RNA is amplified, and the sequence thereof is then determined. Although there is no particular limitation, in order to improve the amplification accuracy, reverse transcription and amplification are preferably performed using multiple types of primers.

The screening method of a library can be used, for example, for
(1) identification of a factor involved in a change in the secretion level of an extracellular vesicle (including a factor involved in a change in the secretion level a vesicle comprising a specific protein);
(2) identification of a factor involved in localization of a specific protein in and on the membrane of an extracellular vesicle;
(3) identification of a factor that influences the half-life or kinetics of an extracellular vesicle in body fluid;
(4) identification of a factor that influences the targeting of an extracellular vesicle to each tissue or each body fluid; or
(5) identification of a factor that influences the targeting of an extracellular vesicle to a specific cell (including a primary cultured cell).

In (1) or (2) above, the target factor can be identified by introducing an expression vector or barcode RNA expressing a barcode RNA into an extracellular vesicle-secreting cell, and comparatively analyzing the barcode RNA collected from the extracellular vesicle secreted by the extracellular vesicle-secreting cell after the introduction and the barcode RNA remaining in the extracellular vesicle-secreting cell. The influence of a specific agent on the change in the secretion level of an extracellular vesicle may be examined by administering the agent to an extracellular vesicle-secreting cell and collecting the extracellular vesicle after a certain period of time.

In (2) above, a factor involved in localization of a specific protein in and on the membrane of an extracellular vesicle can be identified by subjecting the library of collected barcoded extracellular vesicles to precipitation with an antibody (or antibody binding beads) that recognizes the specific protein and analyzing the barcode RNA in the extracellular vesicle having the specific protein on the surface thereof. Here, examples of the factor involved in localization of a specific protein in and on the membrane of an extracellular vesicle include a factor that influences the transcription and translation of the specific protein (e.g., a transcription factor), a factor involved in the post-translational modification of the specific protein (e.g., an enzyme performing glycosylation including GPI anchor binding), and a factor involved in chaperoning (including folding and intracellular transportation) of the specific protein.

The specific protein is preferably a protein existing on the membrane surface or in the membrane of an extracellular vesicle and may be the same as or different from the protein to be used for fusion with the RNA-binding protein. Although there is no particular limitation, examples thereof include tetraspanins, cell adhesion factors such as various integrins, and an interferon-induced transmembrane protein (IFITM3), which is one of the factors that are thought to link the relationship between cellular aging and EVs.

In (3) and (4) above, the target factor can be identified by administering the library of extracellular vesicles prepared in the present disclosure to a subject, isolating tissue or body fluid of the subject after a certain period of time, and detecting the barcode RNA in the isolated tissue or body fluid. In (5) above, the target factor can be identified by administering the library of extracellular vesicles prepared in the present disclosure to a subject cell and detecting a barcode RNA from the cell after a certain period of time. The influence of a specific agent may be examined by administering the agent to a subject (cell) before, after, or simultaneously with administration of the library of extracellular vesicles to the subject (cell) and collecting the extracellular vesicle after a certain period of time.

As the body fluid in (3) and (4) above, body fluid that exists in the body of an animal and fills the spaces between tissues, body cavities, or tubes and circulatory system spreading throughout the body and body fluid that is secreted and excreted inside and outside the body, such as saliva, sweat, semen, and urine, are included. Although there is no particular limitation, examples thereof include blood (including whole blood, serum, and plasma), saliva, urine, amniotic fluid, cerebrospinal fluid, pericardial fluid, pleural fluid, ascites fluid, feces, sweat, and semen.

The tissue in (4) above may be normal tissue (e.g. tissue forming various organs such as nervous tissue, immune tissue, muscle tissue, and digestive tract) or benign or malignant tumor tissue (including blood cancer) of a eukaryote (such as a human, a non-human animal, and a plant) or abnormal tissue such as tissue infected with a pathogen.

The cell in (5) above is a cell that can receive an extracellular vesicle and may be a normal cell (e.g., a differentiated or undifferentiated cell (such as a stem cell)) or an abnormal cell such as a cancer cell of a eukaryote (such as a human, a non-human animal, and a plant) or a cell line established from these cells, a plant cell (such as a callus cell), or a single-celled microorganism (such as an enterobacterium). Identification of such a factor is performed by comparing quantity ratios of barcode RNAs. Although there is no particular limitation, identification may be performed using, as a standard, one having a quantity ratio of 1.5 times or more, 2 times or more, or 3 times or more or 0.3 times or less, 0.5 times or less, or 0.67 times or less. For example, the quantity ratios of barcode RNAs in a DNA library or an RNA library (such as expression vector) used when a library of extracellular vesicles is created and the quantity ratios of barcode RNAs collected from a cell, tissue, body fluid, or an extracellular vesicle may be compared.

Disclosed herein is an extracellular vesicle secretion promoter or secretion inhibitor in vitro or in vivo.

The extracellular vesicle is preferably an extracellular vesicle expressing a tetraspanin (CD63, CD9, CD81, or CD82) on the surface thereof.

Examples of the extracellular vesicle secretion promoter include a PI4KA (phosphatidylinositol 4-kinase alpha) inhibitor, a CYB5B (cytochrome B5 type B) inhibitor, a PIK3C3 (phosphatidylinositol 3-kinase catalytic subunit type 3) inhibitor, a PTPN23 (protein tyrosine phosphatase non-receptor type 23) inhibitor, a PIK3R4 (phosphoinositide-3-kinase regulatory subunit 4) inhibitor, and a METAP1 (methionyl aminopeptidase 1) inhibitor.

Examples of the extracellular vesicle secretion inhibitor include an MMAA (metabolism of cobalamin associated A) inhibitor.

The inhibitor may be an miRNA (including pre-miRNA) or an siRNA (pre-siRNA (e.g., shRNA (small hairpin RNA)) that inhibits the expression of these genes, or may be a low molecular weight compound (a molecular weight of 2000 or less, preferably a molecular weight of 1000 or less, and more preferably a molecular weight of 600 or less), an aptamer, or an antibody (including a binding fragment) that binds to these gene products to inhibit their functions.

The inhibitor of PI4KA (phosphatidylinositol 4-kinase alpha), which is a low molecular weight compound, is preferably GSK-A1 (5-(2-amino-1-(4-morpholinophenyl)-1H-benzo[d]imidazol-6-yl)-N-(2-fluorophenyl)-2-methoxypyridine-3-sulfonamide).

Liquid biopsy is technology for diagnosis or prediction of therapeutic effect mainly in the area of cancer using body fluid samples, such as blood (including whole blood, serum, and plasma), saliva, urine, amniotic fluid, cerebrospinal fluid, pericardial fluid, pleural fluid, ascites fluid, and feces, instead of conventional biopsy collecting tumor tissue with an endoscope or a needle. Research for diagnosis and prediction of therapeutic effect is being performed by detecting a circulating free DNA, a circulating tumor DNA, a circulating free RNA, an extracellular vesicle, or the like that exists in body fluid, and the extracellular vesicle secretion promoter according to the present disclosure can increase the accuracy of the diagnosis and prediction of therapeutic effect through liquid biopsy by promoting secretion of an extracellular vesicle that is secreted from a cancer cell or the like.

The extracellular vesicle secretion inhibitor can be applied to cancer therapy.

Inhibition of secretion of an extracellular vesicle not only prevents cancer metastasis but also can prevent the primary lesion from programming the circumference of itself by secretion of a small EV and influencing the primary lesion itself.

### EXAMPLES

### A. Material and method

### <Fusion protein expression vector>

1. CD63-L7Ae
   A vector plasmid (pRK320) expressing CD63-L7Ae fusion protein (SEQ ID NO: 6) was produced by introducing a sequence encoding CD63-L7Ae fusion protein under an EF-1α promoter of a pSBbi-GH vector (Addgene, plasmid #60514: hygromycin resistance gene + EGFP co-expression type).
2. CD63-MS2
   A vector plasmid (pKK47: pSBbi-GH CD63-MS2) expressing CD63-MS2 fusion protein (SEQ ID NO: 7) was produced by introducing a sequence encoding CD63-MS2 fusion protein at SfiI restriction enzyme recognition site under an EF-1α promoter of pSBbi-GH vector (Addgene, plasmid #60514: hygromycin resistance gene + EGFP co-expression type).
3. CD63-dCas9
   A vector plasmid (pKK60: pSBbi-GH CD63-dCas9) expressing CD63-dCas9 fusion protein (SEQ ID NO: 8) was produced by introducing a sequence encoding CD63-dCas9 fusion protein at SfiI restriction enzyme recognition site under an EF-1α promoter of pSBbi-GH vector (Addgene, plasmid #60514: hygromycin resistance gene + EGFP co-expression type).
4. CD9-dCas9
   A vector plasmid (pKK106: pSBbi-GH CD9-dCas9) expressing CD9-dCas9 fusion protein (SEQ ID NO: 9) was produced by introducing a sequence encoding CD9-dCas9 fusion protein at SfiI restriction enzyme recognition site under an EF-1α promoter of pSBbi-GH vector (Addgene, plasmid #60514: hygromycin resistance gene + EGFP co-expression type).

### <CRISPR vector>

1. Cas9 (for knockout)
   A vector plasmid (pRK300: pSBbi-RB Cas9) expressing SpCas9-NLS-FLAG (SEQ ID NO: 10) was produced by introducing a sequence of Cas9 fusion protein at SfiI restriction enzyme recognition site under an EF-1α promoter of pSBbi-RB vector (Addgene, plasmid #60522: blasticidin resistance gene + RFP co-expression type).
2. dCas9-VPR (for expression enhancement)
   A vector plasmid (pKK56: pSBbi-RB dCas9-VPR) expressing dCas9-VPR fusion protein (SEQ ID NO: 11) was produced by introducing a sequence encoding dCas9-VPR fusion protein under an EF-1α promoter of pSBbi-RB vector (Addgene, plasmid #60522: blasticidin resistance gene + RFP co-expression type).

### <Generation of EV-producing cell>

HEK293T cells were transfected with a fusion protein expression vector (pRK320: CD63-L7Ae, pKK47: CD63-MS2, or pKK60: CD63-dCas9) and, according to the need, a CRISPR vector (pRK300 or pKK56), and drug resistance selection was performed to establish each stable expression cell line.

### <Library for each fusion protein>

1. Library for CD63-L7Ae (case for CRISPRa (expression amplification))
   A backbone sequence (SEQ ID NO: 12) of a gRNA comprising L7Ae recognition sequence site (C/Dbox) was generated by oligo annealing and was cloned to BlpI-XhoI site of pCRISPRia-v2 (Addgene, plasmid #84832). A BlpI-NheI sequence comprising L7Ae recognition sequence site (C/Dbox) and gRNA backbone region was cut out from the resulting plasmid and was cloned in the restriction enzyme site of Addgene Membrane Proteins - gRNA pooled library (1.2 × 10⁴ types) (Addgene, plasmid #83976) of Human Subpooled CRISPRi-v2 Libraries series or Addgene Membrane Proteins - gRNA pooled library (1.2 × 10⁴ types) (Addgene, plasmid #83985) of Human Subpooled CRISPRa-v2 Libraries series to create a library vector for transcribing a sequence recognized by L7Ae protein and an RNA bound to the gRNA.
2(1). Library for CD63-MS2 (case for CRISPRa (expression amplification))
   A backbone sequence of a gRNA comprising MS2 recognition sequence site (MS2box × 2) was amplified in a form comprising BlpI and XhoI recognition sequences at both ends, respectively, by PCR using sgRNA(MS2) cloning backbone (Addgene, plasmid #61424) as a template, and was cloned in the BlpI-XhoI site of pCRISPRia-v2 (Addgene, plasmid #84832). A BlpI-NheI fragment comprising MS2 recognition sequence site (MS2box × 2) and gRNA backbone region was cut out from the resulting plasmid (pKK66) and was cloned in the restriction enzyme recognition site of Addgene Membrane proteins - gRNA pooled library (1.3 × 10⁴ types) (Addgene, plasmid #83976) of Human Subpooled CRISPRi-v2 Libraries series to create a vector library for transcribing a gRNA comprising a sequence recognized by MS2 protein.

In addition, (one or two) vectors expressing gRNA for each of CD274, CD47, CD55, CD59, CD81, ICAM1, ITGAL, LRP1, and IL1B were respectively produced.

The sequence encoding the gRNA comprised in each vector is shown in the following Table 1.

**[Table 1]**

| SEQ ID NO | gRNA | Sequence |
|---|---|---|
| 18 | CD274_gRNA | CTATACACAGCTTTATTCCT |
| 19 | CD274_gRNA | CTGACCTTCGGTGAAATCGG |
| 20 | CD47_gRNA | CAGGACGTGACCTGGAAGCG |
| 21 | CD47_gRNA | GCGTGCCACCGCCCTGGAGC |
| 22 | CD55_gRNA | AAACGGGGTGTAAACGGGAG |
| 23 | CD55_gRNA | GAACTCACGTGCGGGCAGCA |
| 24 | CD59_gRNA | ACCCCAGGGAACTGAAAGTT |
| 25 | CD59_gRNA | CAGGTTCTGAGAAGGCCGGG |
| 26 | CD81_gRNA | CCGCCAGGGCGCGCAGCCGA |
| 27 | ICAM1_gRNA | CAGTTTACCGCTTTGTGAAA |
| 28 | ICAM1_gRNA | CCTCCCTCTCCAGCTTACGC |
| 29 | IL1B_gRNA | AAAAACAGCGAGGGAGAAAC |
| 30 | ITGAL_gRNA | GACAGTCTCCACTGCTGTCA |
| 31 | ITGAL_gRNA | GGCTCTGTAAAGCTCAGCAG |
| 32 | LRP1_gRNA | ATCGGCGCATGCGCACTCAC |
| 33 | LRP1_gRNA | CTGCCCGGGGGAGGGCGACG |

### 2(2). Library for CD63-MS2 (library presenting random peptide in extracellular region of Lamp2b)

A bidirectional promoter (amplified from pSBbi-RB Addgene #60522) sequence, an MS2 recognition sequence × 3, a sequence encoding RVGLamp2b (synthesized by modifying a sequence so as to comprise a desired restriction enzyme recognition sequence from pcDNA GNSTM-3-RVG-10-Lamp2b-HA, Addgene, plasmid #71294), and a sequence encoding bGH poly A were introduced in the site cleaved with NheI and SbfI (excision of the part sandwiched by sequences encoding cPPT and PuroR) using pCRISPRia-v2 (Addgene, plasmid #84832) as a backbone. On this occasion, the RPBSA promoter in the bidirectional promoter was introduced in the forward direction with respect to PuroR, and the EF1a promoter, MS2 recognition sequence × 3, RVGLamp2b, and bGH poly A were in the reverse direction.

Subsequently, the part encoding RVG peptide was excised by restriction enzyme digestion, and a synthetic oligonucleotide library (NDT codon set: 12 × 4 = 20736 types) encoding random peptides (4-mer) was introduced instead to create a library (pSF63) for expressing random peptide-Lamp2b fusion proteins (SEQ ID NO: 34).

### 3. Library for CD63-dCas9

As a library for CD63-dCas9, an existing gRNA library can be used as it is.

In this Example, in addition to the Addgene Membrane proteins - gRNA pooled library (1.3 × 10⁴ types) (Addgene #83976, for CRISPRa) of Human Subpooled CRISPRa-v2 Libraries series, Human CRISPR Knockout Pooled Library (GeCKOv2) (Addgene, Pooled Library #1000000048) and Bassik Human CRISPR Knockout library (NPL 14), Drug Targets, Kinases, phosphatases (DTKP library) (10 gRNAs/gene, 24569 gRNAs in total (2323 target genes), Addgene, Pooled library # 101927) (both were for CRISPR) were used.

Each plasmid library or a gRNA plasmid for each individual gene, lentiviral packaging plasmid psPAX2 (Addgene, plasmid #122260), and pMD2.G (Addgene, plasmid #12259) were transfected to Lenti-X (registered trademark) 293T cells (Takara Bio Inc., Japan). After culturing for 6 to 16 hours, the medium was replaced, the culture supernatant after culturing for 48 hours was subjected to filtration, and a solution comprising lentivirus was collected. EV-producing cells were infected with the collected lentivirus, and the titer (MOI) of the lentivirus was determined by monitoring a fluorescent marker that was separately encoded in a library expression cassette or by cell viability assay using an antibiotic-resistant gene separately encoded in a library expression cassette.

### <Lentivirus infection and EV production>

EV-producing cells were infected with lentivirus according to the measured titer. After the infection, the cells were cultured for 4 days or more in the presence of puromycin.

Subsequently, the culture medium was replaced by OptiMEM (registered trademark) medium (Thermo Fisher Scientific K.K., Japan), and after culturing for 48 hours, the culture supernatant comprising extracellular vesicles was collected. Subsequently, the supernatant was centrifuged at 300G for 5 minutes and then at 1500G for 10 minutes and was passed through a 0.22 µm filter to remove cells and cell debris. Subsequently, the extracellular vesicles were purified and concentrated from the supernatant by ultracentrifugation.

### <Sequence analysis method of barcode RNA>

### 1. RNA derived from gRNA library for CD63-L7Ae

RNAs were extracted from the collected extracellular vesicles or EV-producing cells using TRIzol and were reverse-transcribed using reverse transcription primer AAAGCACCGACTCGGTGCCAC (SEQ ID NO: 14) and reverse transcription enzyme having template switching activity in the presence of LNA (2'-4' bridged nucleic acid, AAGCAGTGGTATCAACGCAGAGTACrGrG+G) (SEQ ID NO: 13: the nucleotide with "r" is an RNA nucleotide, and the nucleotide with "+" is an LNA nucleotide).

This reverse transcription product was amplified by PCR using an adapter sequence for next generation sequencers and an oligo DNA having added thereto a barcode for sample multiplexing as a Fw primer and a Rev primer, respectively. The amplified DNA was analyzed using Ion Proton or Illumina Hiseq X Ten to determine the sequence of each gRNA, and the quantity ratio thereof was calculated.

### 2(1). RNA derived from gRNA library for CD63-MS2

RNAs were extracted from the collected extracellular vesicles or EV-producing cells using TRIzol and were reverse-transcribed using reverse transcription primer AAAGCACCGACTCGGTGCCAC (SEQ ID NO: 14) and reverse transcription enzyme having template switching activity in the presence of LNA (SEQ ID NO: 13). This reverse transcription product was amplified by PCR using an adapter sequence for next generation sequencers and an oligo DNA having added thereto a barcode for sample multiplexing as a Fw primer and a Rev primer, respectively.

The amplified DNA was analyzed using Ion Proton or Illumina Hiseq X Ten to determine the sequence of each gRNA, and the quantity ratio thereof was calculated.

### 2(2). RNA derived from random peptide-Lamp2b library for CD63-MS2

RNAs were extracted from the collected extracellular vesicles or EV-producing cells using TRIzol and were reverse-transcribed using reverse transcription primer attttgcataaaggcaagtgg (SEQ ID NO: 15) and a reverse transcription enzyme, followed by PCR amplification with oligo DNAs having added thereto adapters for next generation sequencers as a Fw primer and a Rev primer, respectively. Analysis with Ion Proton was performed, and the quantity ratio was calculated.

### 3. RNA derived from library for CD63-dCas9 (Addgene, case of using Pooled Library #83976, #1000000048, or # 101927)

RNAs were extracted from the collected extracellular vesicles using TRIzol and were reverse-transcribed using reverse transcription primer TTTTTCAAGTTGATAACGGACTAGCC (SEQ ID NO: 16) and a reverse transcription enzyme having template switching activity in the presence of LNA (2'-4' bridged nucleic acid, AAGCAGTGGTATCAACGCAGAGTACrGrG+G) (SEQ ID NO: 13). This reverse transcription product was amplified by PCR using an adapter sequence for next generation sequencers and an oligo DNA having added thereto a barcode for sample multiplexing as a Fw primer and a Rev primer, respectively. The amplified DNA was analyzed using Ion Proton or Illumina Hiseq X Ten to determine the sequence of each gRNA, and the quantity ratio thereof was calculated.

### B. Evaluation of barcoded CD63-MS2-expressing extracellular vesicle

In order to examine the retentivity in blood of CD63-MS2-expressing extracellular vesicle, the following experiment was performed.

EV-producing cells for CD63-MS2 (HEK293T CD63-MS2, dCas9-VPR-expressing line) were transfected with (one or two) vectors expressing gRNA for each of CD274, CD47, CD55, CD59, CD81, ICAM1, ITGAL, LRP1, and IL1B, respectively, and the culture supernatants comprising extracellular vesicles were collected and mixed.

Extracellular vesicles were purified from the collected culture supernatant by ultracentrifugation and were suspended in PBS at about 1 × 10¹¹ to 10¹² vesicles/mL in total.

The prepared EV solution (100 µL) was injected intravenously in the tail of a Jcl:ICR mouse.

The mouse was euthanatized with CO₂ 2 minutes after intravenous injection, whole blood was collected from the mouse, serum was acquired using a microtainer blood-collecting vessel (Becton Dickinson and Company), and extracellular vesicles in the serum were isolated using Total Exosome Isolation Reagent (Thermo Fisher Scientific K.K., Japan). RNAs were extracted from the isolated extracellular vesicles using a TRIzol reagent, reverse transcription and amplification were performed to determine the sequence of each gRNA, and the quantity ratio thereof was calculated (t = 2 min).

As a control (t = 0), the prepared EV solution before injection to a mouse was subjected to the same treatment as above except EV isolation from serum by the total exosome isolation reagent, the sequence of each gRNA was determined, and the quantity ratio thereof was calculated. Furthermore, the amount of each gRNA collected from blood was calculated by setting the control as 1.

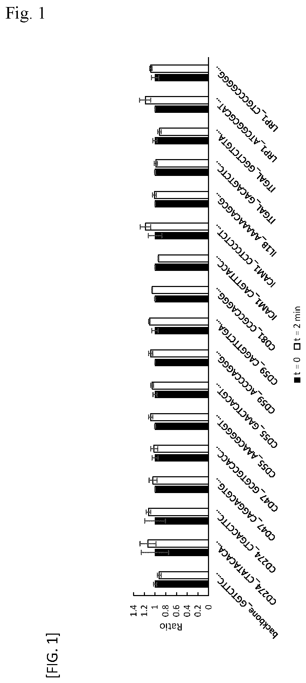

As a result, the 17 gRNAs used were all detected, and the quantity ratios thereof were also stably detected (Fig. 1).

### C. CD63-MS2-expressing EV library and CD63-dCas9-expressing EV library

Regarding the CD63-MS2-expressing EV library (for gRNA), a gRNA library including MS2box in the backbone was created by performing cloning as described as above using Addgene #83985, Human Subpooled CRISPRa-v2 Libraries Membrane proteins - gRNA pooled library (1.3 × 10⁴ types), and the created library was packaged in lentivirus and was infected to EV-producing cells (HEK293T CD63-MS2, dCas9-VPR stable expression cell line).

Regarding the CD63-dCas9-expressing EV library, a library of Addgene #83985 was directly packaged in lentivirus and was infected to EV-producing cells (HEK293T CD63-dCas9, dCas9-VPR stable expression cell line).

The cells after infection were cultured under the same conditions, and extracellular vesicles were collected from the culture supernatant.

RNAs were extracted from the collected extracellular vesicles and were reverse-transcribed and amplified, the sequences were determined, and the quantity ratios thereof were calculated.

As a control, the sequence of the original gRNA library itself was also determined, and the quantity ratio thereof was calculated.

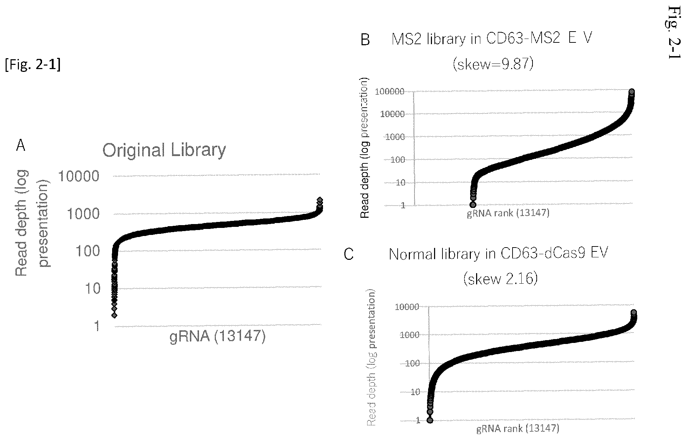

As a result, in the CD63-MS2-expressing EV library, barcoded EVs were detected in 9000 or more gRNAs of 13147 gRNAs (detection rate: 68.4%). Furthermore, in the CD63-dCas9-expressing EV library, barcoded extracellular vesicles were detected in 13120 gRNAs of 13147 gRNAs (detection rate: 99.8%) (Figs. 2A to 2C).

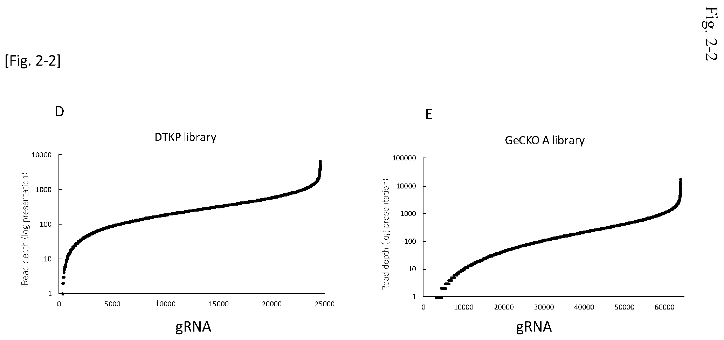

Furthermore, barcoded extracellular vesicles were detected in 60711 gRNAs of 63950 gRNAs (detection rate: 94.9%) in the CD63-dCas9-expressing EV library using Gecko V2A (Addgene #1000000048) library (Fig. 2D), and barcoded extracellular vesicles were detected in 24285 gRNAs of 24569 gRNAs (detection rate: 98.8%) in the CD63-dCas9-expressing EV library using DTKP library (Addgene #101927) (Fig. 2E).

Regarding CD63-MS2-expressing EV library (for random peptide-Lamp2b), the plasmid library created in <Library for each fusion protein> 2(2) above was packaged in lentivirus and was infected to EV-producing cells (HEK293T CD63-MS2, dCas9-VPR stable expression cell line). The cells after infection were cultured under the same conditions, and extracellular vesicles were collected from the culture supernatant.

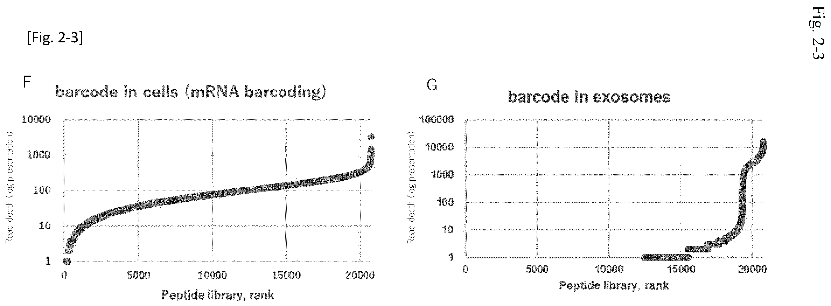

RNAs were extracted from the collected extracellular vesicles and were reverse-transcribed and amplified, the sequences were determined, and the quantity ratios thereof were calculated (Fig. 2G). As a control, RNAs were extracted from the EV-producing cells themselves after collection of the culture supernatant and were reverse-transcribed and amplified. The sequences were determined, and the quantity ratios thereof were calculated (Fig. 2F).

As a result, an EV library expressing random peptides on the surface thereof was successfully created.

### D. Screening for factor extending half-life of exosome in blood using barcoded exosome

Vesicles (3.0 × 10¹⁰ vesicles) of the CD63-dCas9-expressing EV library created in the above C were injected intravenously in the tail of a mouse. Blood was collected from the mouse 30 minutes after injection, and extracellular vesicles were isolated from the collected blood.

RNAs were extracted from the isolated extracellular vesicles, reverse transcription and amplification were performed, and the sequences were determined.

As a control, RNAs were extracted from the CD63-dCas9-expressing EV library before injection and were reverse-transcribed and amplified. The sequences were determined, and the quantity ratios thereof were calculated. Furthermore, the amount of each gRNA collected from blood was calculated by setting the control as 1.

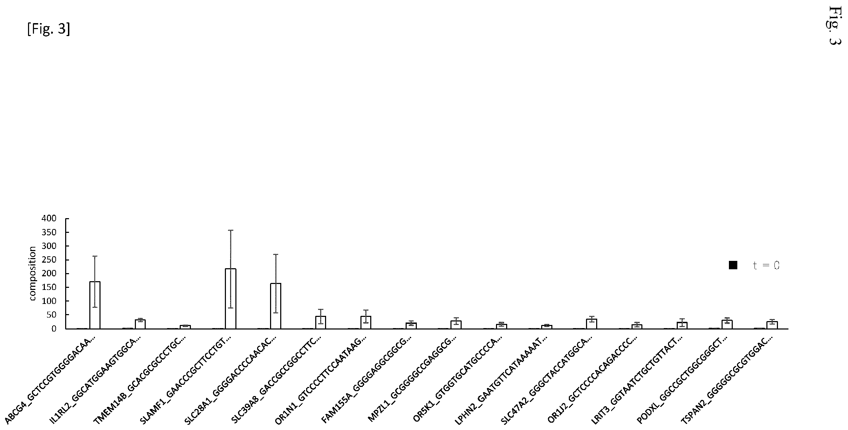

As a result, gRNAs of which the component ratios in blood were significantly increased could be identified (Fig. 3).

The sequences encoding the detected gRNAs are shown in Table 2.

**[Table 2]**

| SEQ ID NO | gRNA | Sequence |
|---|---|---|
| 35 | ABCG4_gRNA | GCTCCGTGGGGACAAGTCCC |
| 36 | IL1RL2_gRNA | GGCATGGAAGTGGCATGACA |
| 37 | TMEM14B_gRNA | GCACGCGCCCTGCATCCCGG |
| 38 | SLAMF1_gRNA | GAACCCGCTTCCTGTATCAC |
| 39 | SLC28A1_gRNA | GGGGACCCCAACACGGTCTC |
| 40 | SLC39A8_gRNA | GACCGCCGGCCTTCCCTCCG |
| 41 | OR1N1_gRNA | GTCCCCTTCCAATAAGGTCA |
| 42 | FAM155A_gRNA | GGGGAGGCGGCGTCAGCAGT |
| 43 | MPZL1_gRNA | GCGGGGCCGAGGCGCCTGGG |
| 44 | OR5K1_gRNA | GTGGTGCATGCCCCATTCAT |
| 45 | LPHN2_gRNA | GAATGTTCATAAAAATCGGA |
| 46 | SLC47A2_gRNA | GGGCTACCATGGCAACTTGT |
| 47 | OR1J2_gRNA | GCTCCCCACAGACCCCCGAT |
| 48 | LRIT3_gRNA | GGTAATCTGCTGTTACTAAA |
| 49 | PODXL_gRNA | GGCCGCTGGCGGGCTCCTGG |
| 50 | TSPAN2_gRNA | GGGGGCGCGTGGACCCCAAG |

### E1. Screening for factor promoting or inhibiting secretion of extracellular vesicle using barcoded extracellular vesicle

HEK293T cells were transfected with a CD63-dCas9 expression vector only and were subjected to drug resistance selection to establish a stable expression cell line (hereinafter, referred to as Cas9(-) EV-producing cell).

HEK293T cells were transfected with a CD63-dCas9 expression vector and a pRK300 plasmid and were subjected to drug resistance selection to establish a stable expression cell line (hereinafter, referred to as Cas9(+) EV-producing cell).

The Cas9(-) EV-producing cell and the Cas9(+) EV-producing cell were infected with lentivirus comprising a DTKP library. After the infection, the cells were cultured for 7 days or more in the presence of puromycin. The culture media were then replaced by OptiMEM (registered trademark) medium (Thermo Fisher Scientific K.K., Japan), and after culturing for 48 hours, the culture supernatants comprising extracellular vesicles were collected. The supernatants were centrifuged at 300 × g for 5 minutes and then at 1500 × g for 10 minutes and were each passed through a 0.22 µm filter to remove cells and cell debris. Subsequently, the extracellular vesicles (Cas9(-) EV and Cas9(+) EV) were purified and concentrated from the cell supernatants by ultracentrifugation.

RNAs were extracted from the collected extracellular vesicles and were reverse-transcribed and amplified, and the sequences were determined and quantitatively measured with a next generation sequencer.

RNAs were extracted also from the cultured EV-producing cells and were reverse-transcribed and amplified, and the sequences were determined and quantitatively measured with a next generation sequencer.

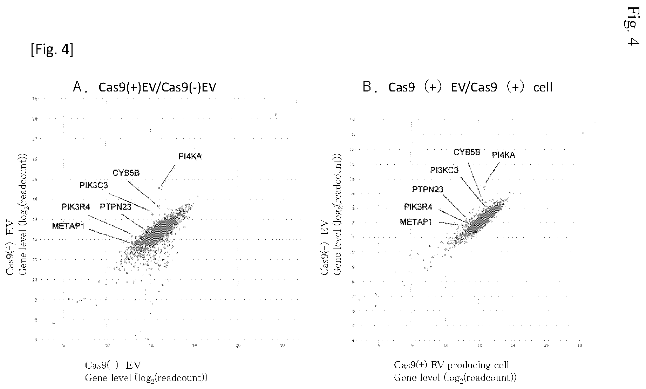

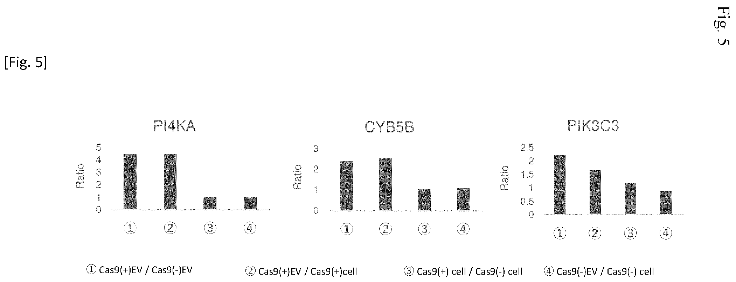

The quantity ratio of the gRNA corresponding to each gene was calculated using CRISPR AnalyzeR (bioRxiv 2017, http://crispr-analyzer.dkfz.de/) for Cas9(-) EV/Cas9(+) EV or Cas9(+) EV/Cas9(+) EV-producing cell (Figs. 4 and 5).

As a result, the secretion levels of extracellular vesicles were increased by knockout of PI4KA, CYB5B, PIK3C3, PTPN23, PIK3R4, and METAP1 (Fig. 4A). This effect did not depend on the number of EV-producing cells expressing each gRNA (Fig. 4B).

### E2. Increase in secretion level of CD63 positive EV by PI4KA inhibitor

A PI4KA inhibitor, GSK-A1, was administered to HEK293T cells expressing CD63-nanoLuc (pDB30), and the amount of secreted extracellular vesicles was measured by luminescence assay (Promega Corporation, Nanoglo luciferase assay system) that measures the nluc activity in a culture supernatant or by nanoparticle tracking analysis (NTA) using Nanosight (Malvern Panalytical Ltd.). As a control, the same experiment was performed without administering GSK-A1, and the EV secretion level at each GSK-A1 concentration was calculated by setting the EV secretion level of the control as 1.

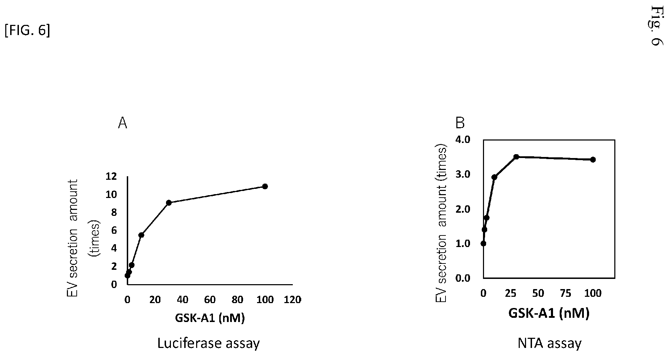

As a result, it was revealed that the secretion level of a CD63 positive extracellular vesicle was increased by addition of GSK-A1 (Fig. 6). This demonstrates that a factor that increases the secretion level of an extracellular vesicle can be identified by a screening method according to the present disclosure.

In addition, the same assay as above was performed using HEK293T cells expressing CD9-dCas9 (SEQ ID NO: 9) as the EV-producing cell. As a result, the secretion level of an exosome comprising a gRNA that knockouts MMAA (metabolism of cobalamin associated A) as a barcode was suppressed (0.62 times compared to that in the cell). In contrast, when HEK293T expressing CD63-dCas9 was used as the EV-producing cell, the secretion level did not change (1.06 times compared to that in the cell). This demonstrates that it is possible to investigate, for example, a difference in EV secretion mechanism of specific population by changing the EV marker to be used.

### Industrial Applicability

The use of the barcoded exosome according to the present disclosure can be utilized for development of an efficient drug delivery system using an exosome, biology research of an exosome (for example, investigation of EV secretion in various cells), and drug discovery research targeting an exosome secretory path (for example, identification of a factor that changes the EV secretion level in a cell-specific manner).

The present disclosure can also be used for analysis of networks via exosomes that transcend the boundaries of living organisms. For example, in the digestive tract of mammals, not only plants (food) but also enterobacteria, pathogenic microorganisms, yeast in food, etc. build relationships such that they constantly interact with each other, and the present disclosure can also be useful for research of exosomes that are generated by a community of these different living organisms.

The extracellular vesicle secretion promoter or inhibitor according to the present disclosure can also be used for analysis of the physiological role of specific population of a specific extracellular vesicle by in vivo or in vitro administration to a subject to promote or inhibit the sub-population of the extracellular vesicle.

## Claims

1. A method of screening for a factor that contributes to stability of an extracellular vesicle in body fluid or a factor that promotes or inhibits secretion of an extracellular vesicle into body fluid,the method comprising:
(1) a step of providing a library comprising multiple types of extracellular vesicles comprising a fusion protein and a barcode RNA bound to the fusion protein, the fusion protein comprising a protein existing in an extracellular vesicle and an RNA-binding protein and the barcode RNA comprising a recognition sequence for the RNA-binding protein; and
(2) a step of detecting barcode RNAs from the extracted RNAs from a body fluid sample obtained from a subject administered with the library comprising multiple types of extracellular vesicles from step 1.

2. The method according to Claim 1, wherein the protein existing in an extracellular vesicle is a tetraspanin such as CD63, CD9, and CD81,or a fragment thereof having an activity for existing in an extracellular vesicle.

3. The method according to Claim 1 or 2, wherein the RNA-binding protein is selected from the group consisting of MS2 or a fragment thereof having RNA-binding activity, CAS or a fragment thereof having RNA-binding activity, L7Ae or a fragment thereof having RNA-binding activity, λ bacteriophage antiterminator protein N or a fragment thereof having RNA-binding activity, and HuR or a fragment thereof having RNA-binding activity.

4. The method according to any one of Claims 1 to 3, wherein the barcode RNA further comprises an mRNA or an ncRNA.

5. A method of screening for a factor that influences efficiency of targeting of an extracellular vesicle to tissue or body fluid, the method comprising:
(1) a step of providing a library comprising multiple types of extracellular vesicles comprising a fusion protein and a barcode RNA bound to the fusion protein, the fusion protein comprising a protein existing in an extracellular vesicle and an RNA-binding protein and the barcode RNA comprising a recognition sequence for the RNA-binding protein; and
(2) a step of detecting barcode RNAs from the extracted RNAs from a tissue sample or body fluid sample obtained from a subject administered with the library comprising multiple types of extracellular vesicles from step 1.

6. The method according to Claim 5, wherein the protein existing in an extracellular vesicle is a tetraspanin such as CD63, CD9, and CD81, or a fragment thereof having an activity for existing in an extracellular vesicle.

7. The method according to Claim 5 or 6, wherein the RNA-binding protein is selected from the group consisting of MS2 or a fragment thereof having RNA-binding activity, CAS or a fragment thereof having RNA-binding activity, L7Ae or a fragment thereof having RNA-binding activity, λ bacteriophage antiterminator protein N or a fragment thereof having RNA-binding activity, and HuR or a fragment thereof having RNA-binding activity.

8. The method according to Claim 7, wherein the barcode RNA further comprises an mRNA or an ncRNA.

9. The method according to Claim 7 or 8, wherein the tissue is selected from the group consisting of tumor tissue, nervous tissue, and immune tissue.

10. An in vitro method of screening for a factor that influences efficiency of targeting of an extracellular vesicle to a cell, the method comprising:
(1) a step of providing multiple types of extracellular vesicles comprising a fusion protein and a barcode RNA bound to the fusion protein, the fusion protein comprising a protein existing in an extracellular vesicle and an RNA-binding protein and the barcode RNA comprising a recognition sequence for the RNA-binding protein;
(2) a step of administering the multiple types of extracellular vesicles to a cell;
(3) a step of extracting RNAs from the cell; and
(4) a step of detecting barcode RNAs from the extracted RNAs.

11. The method according to Claim 10, wherein the protein existing in an extracellular vesicle is a tetraspanin such as CD63, CD9, and CD81, or a fragment thereof having an activity for existing in an extracellular vesicle.

12. The method according to Claim 10 or 11, wherein the RNA-binding protein is selected from the group consisting of MS2 or a fragment thereof having RNA-binding activity, CAS or a fragment thereof having RNA-binding activity, L7Ae or a fragment thereof having RNA-binding activity, λ bacteriophage antiterminator protein N or a fragment thereof having RNA-binding activity, and HuR or a fragment thereof having RNA-binding activity.

13. The method according to any one of Claims 10 to 12, wherein the barcode RNA further comprises an mRNA or an ncRNA.

14. The method according to any one of Claims 10 to 13, wherein the cell is selected from a stem cell, an epithelial cell, an endothelial cell, a fibroblast cell, a cancer cell, an immune cell, a nerve cell, and cell lines established therefrom.

15. The method according to any one of Claims 1 to 9, wherein the method is an in vitro method, optionally wherein the body fluid is selected from the group consisting of blood, saliva, urine, amniotic fluid, cerebrospinal fluid, pericardial fluid, pleural fluid, ascites fluid, feces, sweat, and semen.

## Patentansprüche

1. Verfahren zum Screening nach einem Faktor, der zur Stabilität eines extrazellulären Vesikels in einer Körperflüssigkeit beiträgt, oder nach einem Faktor, der die Sekretion eines extrazellulären Vesikels in eine Körperflüssigkeit fördert oder hemmt, das Verfahren aufweisend:
(1) einen Schritt zum Bereitstellen einer Bibliothek, die mehrere Arten extrazellulärer Vesikel aufweist, welche ein Fusionsprotein und eine an das Fusionsprotein gebundene Barcode-RNA aufweisen, wobei das Fusionsprotein ein in einem extrazellulären Vesikel vorhandenes Protein und ein RNA-bindendes Protein aufweist und die Barcode-RNA eine Erkennungssequenz für das RNA-bindende Protein aufweist; und
(2) einen Schritt zum Nachweis von Barcode-RNAs aus den extrahierten RNAs einer Körperflüssigkeitsprobe, die von einem Probanden gewonnen wurde, dem die Bibliothek, die mehrere Arten extrazellulärer Vesikel aufweist, aus Schritt 1 verabreicht wurde.

2. Verfahren nach Anspruch 1, wobei das in einem extrazellulären Vesikel vorkommende Protein ein Tetraspanin wie CD63, CD9 und CD81, oder ein Fragment davon ist, das über eine Aktivität verfügt, die es ihm ermöglicht, in einem extrazellulären Vesikel vorhanden zu sein.

3. Verfahren nach Anspruch 1 oder 2, wobei das RNA-bindende Protein ausgewählt ist aus der Gruppe bestehend aus MS2 oder einem Fragment davon mit RNA-Bindungsaktivität, CAS oder einem Fragment davon mit RNA-Bindungsaktivität, L7Ae oder einem Fragment davon mit RNA-Bindungsaktivität, dem Bakteriophagen-Antiterminatorprotein N oder einem Fragment davon mit RNA-Bindungsaktivität, und HuR oder einem Fragment davon mit RNA-Bindungsaktivität.

4. Verfahren nach einem der Ansprüche 1 bis 3, wobei die Barcode-RNA ferner eine mRNA oder eine ncRNA aufweist.

5. Verfahren zum Screening nach einem Faktor, der die Effizienz der Zielgerichtetheit eines extrazellulären Vesikels auf Gewebe oder Körperflüssigkeit beeinflusst, das Verfahren aufweisend:
(1) einen Schritt zum Bereitstellen einer Bibliothek, die mehrere Arten extrazellulärer Vesikel aufweist, welche ein Fusionsprotein und eine an das Fusionsprotein gebundene Barcode-RNA aufweisen, wobei das Fusionsprotein ein in einem extrazellulären Vesikel vorhandenes Protein und ein RNA-bindendes Protein aufweist und die Barcode-RNA eine Erkennungssequenz für das RNA-bindende Protein aufweist; und
(2) einen Schritt zum Nachweis von Barcode-RNAs aus den extrahierten RNAs einer Körperflüssigkeitsprobe, die von einem Probanden gewonnen wurde, dem die Bibliothek, die mehrere Arten extrazellulärer Vesikel aufweist, aus Schritt 1 verabreicht wurde.

6. Verfahren nach Anspruch 5, wobei das in einem extrazellulären Vesikel vorkommende Protein ein Tetraspanin wie CD63, CD9 und CD81 oder ein Fragment davon ist, das über eine Aktivität verfügt, die es ihm ermöglicht, in einem extrazellulären Vesikel vorhanden zu sein.

7. Verfahren nach Anspruch 5 oder 6, wobei das RNA-bindende Protein ausgewählt ist aus der Gruppe bestehend aus MS2 oder einem Fragment davon mit RNA-Bindungsaktivität, CAS oder einem Fragment davon mit RNA-Bindungsaktivität, L7Ae oder einem Fragment davon mit RNA-Bindungsaktivität, dem Bakteriophagen-Antiterminatorprotein N oder einem Fragment davon mit RNA-Bindungsaktivität sowie HuR oder einem Fragment davon mit RNA-Bindungsaktivität.

8. Verfahren nach Anspruch 7, wobei die Barcode-RNA ferner eine mRNA oder eine ncRNA aufweist.

9. Verfahren nach Anspruch 7 oder 8, wobei das Gewebe aus der Gruppe ausgewählt ist, die aus Tumorgewebe, Nervengewebe und Immungewebe besteht.

10. *In-vitro*-Verfahren zum Screening nach einem Faktor, der die Effizienz der Zielgerichtetheit eines extrazellulären Vesikels an eine Zelle beeinflusst, das Verfahren aufweisend:
(1) einen Schritt, bei dem mehrerer Arten von extrazellulären Vesikeln bereitgestellt werden, die ein Fusionsprotein und eine an das Fusionsprotein gebundene Barcode-RNA umfassen, wobei das Fusionsprotein ein in einem extrazellulären Vesikel vorhandenes Protein und ein RNA-bindendes Protein aufweist und die Barcode-RNA eine Erkennungssequenz für das RNA-bindende Protein aufweist;
(2) einen Schritt, bei dem die mehreren Arten extrazellulärer Vesikel einer Zelle verabreicht werden;
(3) einen Schritt, bei dem RNA aus der Zelle extrahiert wird; und
(4) ein Schritt, bei dem Barcode-RNAs aus den extrahierten RNAs nachgewiesen werden.

11. Verfahren nach Anspruch 10, wobei das in einem extrazellulären Vesikel vorkommende Protein ein Tetraspanin wie CD63, CD9 und CD81 oder ein Fragment davon ist, das über eine Aktivität verfügt, die es ihm ermöglicht, in einem extrazellulären Vesikel vorhanden zu sein.

12. Verfahren nach Anspruch 10 oder 11, wobei das RNA-bindende Protein ausgewählt ist aus der Gruppe, bestehend aus MS2 oder einem Fragment davon mit RNA-Bindungsaktivität, CAS oder einem Fragment davon mit RNA-Bindungsaktivität, L7Ae oder einem Fragment davon mit RNA-Bindungsaktivität, dem Bakteriophagen-Antiterminatorprotein N oder einem Fragment davon mit RNA-Bindungsaktivität sowie HuR oder einem Fragment davon mit RNA-Bindungsaktivität.

13. Verfahren nach einem der Ansprüche 10 bis 12, wobei die Barcode-RNA ferner eine mRNA oder eine ncRNA aufweist.

14. Verfahren nach einem der Ansprüche 10 bis 13, wobei die Zelle aus einer Stammzelle, einer Epithelzelle, einer Endothelzelle, einer Fibroblastenzelle, einer Krebszelle, einer Immunzelle, einer Nervenzelle und daraus etablierten Zelllinien ausgewählt ist.

15. Verfahren nach einem der Ansprüche 1 bis 9,
wobei es sich bei dem Verfahren um ein *In-vitro*-Verfahren handelt,
wobei die Körperflüssigkeit optional ausgewählt ist aus der Gruppe bestehend aus Blut, Speichel, Urin, Fruchtwasser, Liquor cerebrospinalis, Perikardflüssigkeit, Pleuraflüssigkeit, Aszitesflüssigkeit, Stuhl, Schweiß und Sperma.

## Revendications

1. Procédé de criblage d'un facteur qui contribue à la stabilité d'une vésicule extra-cellulaire dans un fluide corporel ou d'un facteur qui favorise ou inhibe la sécrétion d'une vésicule extra-cellulaire dans un fluide corporel, le procédé comprenant :
(1) une étape consistant à fournir une bibliothèque comprenant de multiples types de vésicules extra-cellulaires comprenant une protéine de fusion et un ARN à code-barres lié à la protéine de fusion, la protéine de fusion comprenant une protéine présente dans une vésicule extra-cellulaire et une protéine de liaison à l'ARN, et l'ARN à code-barres comprenant une séquence de reconnaissance pour la protéine de liaison à l'ARN ; et
(2) une étape consistant à détecter des ARN à code-barres parmi les ARN extraits d'un échantillon de fluide corporel obtenu à partir d'un sujet auquel a été administrée la bibliothèque comprenant de multiples types de vésicules extra-cellulaires provenant de l'étape 1.

2. Procédé selon la revendication 1, dans lequel la protéine présente dans une vésicule extra-cellulaire est une tétraspanine telle que CD63, CD9 et CD81, ou un fragment de celle-ci ayant une activité lui permettant d'être présente dans une vésicule extra-cellulaire.

3. Procédé selon la revendication 1 ou 2, dans lequel la protéine de liaison à l'ARN est choisie dans le groupe constitué par MS2 ou un fragment de celle-ci ayant une activité de liaison à l'ARN, CAS ou un fragment de celle-ci ayant une activité de liaison à l'ARN, L7Ae ou un fragment de celle-ci ayant une activité de liaison à l'ARN, la protéine antiterminatrice N du bactériophage λ ou un fragment de celle-ci ayant une activité de liaison à l'ARN, et HuR ou un fragment de celle-ci ayant une activité de liaison à l'ARN.

4. Procédé selon l'une quelconque des revendications 1 à 3, dans lequel l'ARN à code-barres comprend en outre un ARNm ou un ARNnc.

5. Procédé de criblage d'un facteur qui influence l'efficacité du ciblage d'une vésicule extra-cellulaire vers un tissu ou un fluide corporel, le procédé comprenant :
(1) une étape consistant à fournir une bibliothèque comprenant de multiples types de vésicules extra-cellulaires comprenant une protéine de fusion et un ARN à code-barres lié à la protéine de fusion, la protéine de fusion comprenant une protéine présente dans une vésicule extra-cellulaire et une protéine de liaison à l'ARN, et l'ARN à code-barres comprenant une séquence de reconnaissance pour la protéine de liaison à l'ARN ; et
(2) une étape consistant à détecter des ARN à code-barres parmi les ARN extraits d'un échantillon de tissu ou d'un échantillon de fluide corporel obtenu à partir d'un sujet auquel a été administrée la bibliothèque comprenant de multiples types de vésicules extra-cellulaires provenant de l'étape 1.

6. Procédé selon la revendication 5, dans lequel la protéine présente dans une vésicule extra-cellulaire est une tétraspanine telle que CD63, CD9 et CD81, ou un fragment de celle-ci ayant une activité lui permettant d'être présente dans une vésicule extra-cellulaire.

7. Procédé selon la revendication 5 ou 6, dans lequel la protéine de liaison à l'ARN est choisie dans le groupe constitué par MS2 ou un fragment de celle-ci ayant une activité de liaison à l'ARN, CAS ou un fragment de celle-ci ayant une activité de liaison à l'ARN, L7Ae ou un fragment de celle-ci ayant une activité de liaison à l'ARN, la protéine antiterminatrice N du bactériophage λ ou un fragment de celle-ci ayant une activité de liaison à l'ARN, et HuR ou un fragment de celle-ci ayant une activité de liaison à l'ARN.

8. Procédé selon la revendication 7, dans lequel l'ARN à code-barres comprend en outre un ARNm ou un ARNnc.

9. Procédé selon la revendication 7 ou 8, dans lequel le tissu est choisi dans le groupe constitué par un tissu tumoral, un tissu nerveux et un tissu immunitaire.

10. Procédé in vitro de criblage d'un facteur qui influence l'efficacité du ciblage d'une vésicule extra-cellulaire vers une cellule, le procédé comprenant :
(1) une étape consistant à fournir de multiples types de vésicules extra-cellulaires comprenant une protéine de fusion et un ARN à code-barres lié à la protéine de fusion, la protéine de fusion comprenant une protéine présente dans une vésicule extra-cellulaire et une protéine de liaison à l'ARN, et l'ARN à code-barres comprenant une séquence de reconnaissance pour la protéine de liaison à l'ARN ;
(2) une étape consistant à administrer les multiples types de vésicules extra-cellulaires à une cellule ;
(3) une étape consistant à extraire des ARN de la cellule ; et
(4) une étape consistant à détecter des ARN à code-barres parmi les ARN extraits.

11. Procédé selon la revendication 10, dans lequel la protéine présente dans une vésicule extra-cellulaire est une tétraspanine telle que CD63, CD9 et CD81, ou un fragment de celle-ci ayant une activité lui permettant d'être présente dans une vésicule extra-cellulaire.

12. Procédé selon la revendication 10 ou 11, dans lequel la protéine de liaison à l'ARN est choisie dans le groupe constitué par MS2 ou un fragment de celle-ci ayant une activité de liaison à l'ARN, CAS ou un fragment de celle-ci ayant une activité de liaison à l'ARN, L7Ae ou un fragment de celle-ci ayant une activité de liaison à l'ARN, la protéine antiterminatrice N du bactériophage λ ou un fragment de celle-ci ayant une activité de liaison à l'ARN, et HuR ou un fragment de celle-ci ayant une activité de liaison à l'ARN.

13. Procédé selon l'une quelconque des revendications 10 à 12, dans lequel l'ARN à code-barres comprend en outre un ARNm ou un ARNnc.

14. Procédé selon l'une quelconque des revendications 10 à 13, dans lequel la cellule est choisie parmi une cellule-souche, une cellule épithéliale, une cellule endothéliale, un fibroblaste, une cellule cancéreuse, une cellule immunitaire, une cellule nerveuse, et des lignées cellulaires établies à partir de celles-ci.

15. Procédé selon l'une quelconque des revendications 1 à 9, dans lequel le procédé est un procédé in vitro, dans lequel, de manière optionnelle, le fluide corporel est choisi dans le groupe constitué par le sang, la salive, l'urine, le liquide amniotique, le liquide céphalorachidien, le liquide péricardique, le liquide pleural, le liquide d'ascite, les fèces, la sueur et le sperme.
